# EUROPEAN PATENT SPECIFICATION

(11) **EP 0 612 353 B1**
(45) Date of publication and mention of the grant of the patent: **31.03.1999**
(21) Application number: 92920292.7
(22) Date of filing: 16.09.1992
(51) Int. Cl.: C12Q 1/68

(54) **DETECTION METHOD FOR C-RAF-1 GENES**
NACHWEISMETHODEN FÜR C-Raf-1-GENES.5
PROCEDE DE DETECTION DE GENES C-RAF-1

(30) Priority: 16.09.1991 US 759738
(43) Date of publication of application: 31.08.1994
(73) Proprietor: THE GOVERNMENT OF THE UNITED STATES OF AMERICA, as represented by THE SECRETARY OF THE DEPARTMENT OF HEALTH AND HUMAN SERVICES, Rockville, MD 20852-3804 (US)
(72) Inventor: RAPP, Ulf, R., Washington, DC 20015 (US); STORM, Stephen, M., Frederick, MD 21702 (US)
(74) Representative: Perry, Robert Edward
(86) International application number: US9207817
(87) International publication number: WO9306248

(56) References cited:
- WO-A-90/09456
- WO-A-91/05064
- WO-A-91/12343
- Chemical Abstracts, volume 104, no. 19, 12 May 1986, (Columbus, Ohio, US), Bonner Tom I. et al. : "The complete coding sequence of the human raf oncogene and the corresponding structure of the c-raf-1 gene ", see page 141, abstract
- 162785c, & Nucleic Acids Res. 1986, 14( 2), 1009-101

### SEQUENCE LISTING

(1) GENERAL INFORMATION:
   (i) APPLICANT: The Government of the United States of America as represented by the Secretary of the Department of Health and Human Services
   (ii) TITLE OF INVENTION: DETECTION METHOD FOR C-RAF-1 GENES
   (iii) NUMBER OF SEQUENCES: 12
   (iv) CORRESPONDENCE ADDRESS:
      (A) ADDRESSEE:
      (B) STREET: 200 Independence Avenue
      (C) CITY: Washington
      (D) STATE: DC
      (E) COUNTRY: USA
      (F) ZIP: 20201
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
(2) INFORMATION FOR SEQ ID NO:1:
   (i) SEQUENCE CHARACTERISTICS:
      (A) LENGTH: 648 amino acids
      (B) TYPE: amino acid
      (D) TOPOLOGY: linear
   (xi) SEQUENCE DESCRIPTION: SEQ ID NO:1:
   Met Glu His Ile Gln Gly Ala Trp Lys Thr Ile Ser Asn Gly Phe Gly
   1               5                   10                  15
   Leu Lys Asp Ala Val Phe Asp Gly Ser Ser Cys Ile Ser Pro Thr Ile
   20 25 30
   Val Gln Gln Phe Gly Tyr Gln Arg Arg Ala Ser Asp Asp Gly Lys Leu
   35 40 45
   Thr Asp Ser Ser Lys Thr Ser Asn Thr Ile Arg Val Phe Leu Pro Asn
   50 55 60
   Lys Gln Arg Thr Val Val Asn Val Arg Asn Gly Met Ser Leu His Asp
   65 70 75 80
   Cys Leu Met Lys Ala Leu Lys Val Arg Gly Leu Gln Pro Glu Cys Cys
   85 90 95
   Ala Val Phe Arg Leu Leu Gln Glu His Lys Gly Lys Lys Ala Arg Leu
   100 105 110
   Asp Trp Asn Thr Asp Ala Ala Ser Leu Ile Gly Glu Glu Leu Gln Val
   115 120 125
   Asp Phe Leu Asp His Val Pro Ile Thr Thr His Asn Phe Ala Arg Lys
   130 135 140
   Thr Phe Leu Lys Leu Ala Phe Cys Asp Ile Cys Gln Lys Phe Leu Leu
   145 150 155 160
   Asn Gly Phe Arg Cys Gln Thr Cys Gly Tyr Lys Phe His Glu His Cys
   165 170 175
   Ser Thr Lys Val Pro Thr Met Cys Val Asp Trp Ser Asn Ile Arg Gln
   180 185 190
   Leu Leu Leu Phe Pro Asn Ser Thr Val Gly Asp Ser Gly Val Pro Ala
   195 200 205
   Pro Pro Ser Phe Pro Met Arg Arg Met Arg Glu Ser Val Ser Arg Met
   210 215 220
   Pro Ala Ser Ser Gln His Arg Tyr Ser Thr Pro His Ala Phe Thr Phe
   225 230 235 240
   Asn Thr Ser Ser Pro Ser Ser Glu Gly Ser Leu Ser Gln Arg Gln Arg
   245 250 255
   Ser Thr Ser Thr Pro Asn Val His Met Val Ser Thr Thr Leu His Val
   260 265 270
   Asp Ser Arg Met Ile Glu Asp Ala Ile Arg Ser His Ser Glu Ser Ala
   275 280 285
   Ser Pro Ser Ala Leu Ser Ser Ser Pro Asn Asn Leu Ser Pro Thr Gly
   290 295 300
   Trp Ser Gln Pro Lys Thr Pro Val Pro Ala Gln Arg Glu Arg Ala Pro
   305 310 315 320
   Gly Ser Gly Thr Gln Gln Lys Asn Lys Ile Arg Pro Arg Gly Gln Arg
   325 330 335
   Asp Ser Ser Tyr Tyr Trp Glu Ile Glu Ala Ser Glu Val Met Leu Ser
   340 345 350
   Thr Arg Ile Gly Ser Gly Ser Phe Gly Thr Val Tyr Lys Gly Lys Trp
   355 360 365
   His Gly Asp Val Ala Val Lys Ile Leu Lys Val Val Asp Pro Thr Pro
   370 375 380
   Glu Gln Leu Gln Ala Phe Arg Asn Glu Val Ala Val Leu Arg Lys Thr
   385 390 395 400
   Arg His Val Asn Ile Leu Leu Phe Met Gly Tyr Met Thr Lys Asp Asn
   405 410 415
   Leu Ala Ile Val Thr Gln Trp Cys Glu Gly Ser Ser Leu Tyr Lys His
   420 425 430
   Leu His Val Gln Glu Thr Lys Phe Gln Met Phe Gln Leu Ile Asp Ile
   435 440 445
   Ala Arg Gln Thr Ala Gln Gly Met Asp Tyr Leu His Ala Lys Asn Ile
   450 455 460
   Ile His Arg Asp Met Lys Ser Asn Asn Ile Phe Leu His Glu Gly Leu
   465 470 475 480
   Thr Val Lys Ile Gly Asp Phe Gly Leu Ala Thr Val Lys Ser Arg Trp
   485 490 495
   Ser Gly Ser Gln Gln Val Glu Gln Pro Thr Gly Ser Val Leu Trp Met
   500 505 510
   Ala Pro Glu Val Ile Arg Met Gln Asp Asp Asn Pro Phe Ser Phe Gln
   515 520 525
   Ser Asp Val Tyr Ser Tyr Gly Ile Val Leu Tyr Glu Leu Met Ala Gly
   530 535 540
   Glu Leu Pro Tyr Ala His Ile Asn Asn Arg Asp Gln Ile Ile Phe Met
   545 550 555 560
   Val Gly Arg Gly Tyr Ala Ser Pro Asp Leu Ser Arg Leu Tyr Lys Asn
   565 570 575
   Cys Pro Lys Ala Met Lys Arg Leu Val Ala Asp Cys Val Lys Lys Val
   580 585 590
   Lys Glu Glu Arg Pro Leu Phe Pro Gln Ile Leu Ser Ser Ile Glu Leu
   595 600 605
   Leu Gln His Ser Leu Pro Lys Ile Asn Arg Ser Ala Ser Glu Pro Ser
   610 615 620
   Leu His Arg Ala Ala His Thr Glu Asp Ile Asn Ala Cys Thr Leu Thr
   625 630 635 640
   Thr Ser Pro Arg Leu Pro Val Phe
   645
(2) INFORMATION FOR SEQ ID NO:2:
   (i) SEQUENCE CHARACTERISTICS:
      (A) LENGTH: 648 amino acids
      (B) TYPE: amino acid
      (D) TOPOLOGY: linear
   (xi) SEQUENCE DESCRIPTION: SEQ ID NO:2:
   Met Glu His Ile Gln Gly Ala Trp Lys Thr Ile Ser Asn Gly Phe Gly
   1 5 10 15
   Leu Lys Asp Ala Val Phe Asp Gly Ser Ser Cys Ile Ser Pro Thr Ile
   20 25 30
   Val Gln Gln Phe Gly Tyr Gln Arg Arg Ala Ser Asp Asp Gly Lys Leu
   35 40 45
   Thr Asp Ser Ser Lys Thr Ser Asn Thr Ile Arg Val Phe Leu Pro Asn
   50 55 60
   Lys Gln Arg Thr Val Val Asn Val Arg Asn Gly Met Ser Leu His Asp
   65 70 75 80
   Cys Leu Met Lys Ala Leu Lys Val Arg Gly Leu Gln Pro Glu Cys Cys
   85 90 95
   Ala Val Phe Arg Leu Leu Gln Glu His Lys Gly Lys Lys Ala Arg Leu
   100 105 110
   Asp Trp Asn Thr Asp Ala Ala Ser Leu Ile Gly Glu Glu Leu Gln Val
   115 120 125
   Asp Phe Leu Asp His Val Pro Ile Thr Thr His Asn Phe Ala Arg Lys
   130 135 140
   Thr Phe Leu Lys Leu Ala Phe Cys Asp Ile Cys Gln Lys Phe Leu Leu
   145 150 155 160
   Asn Gly Phe Arg Cys Gln Thr Cys Gly Tyr Lys Phe His Glu His Cys
   165 170 175
   Ser Thr Lys Val Pro Thr Met Cys Val Asp Trp Ser Asn Ile Arg Gln
   180 185 190
   Leu Leu Leu Phe Pro Asn Ser Thr Val Gly Asp Ser Gly Val Pro Ala
   195 200 205
   Pro Pro Ser Phe Pro Met Arg Arg Met Arg Glu Ser Val Ser Arg Met
   210 215 220
   Pro Ala Ser Ser Gln His Arg Tyr Ser Thr Pro His Ala Phe Thr Phe
   225 230 235 240
   Asn Thr Ser Ser Pro Ser Ser Glu Gly Ser Leu Ser Gln Arg Gln Arg
   245 250 255
   Ser Thr Ser Thr Pro Asn Val His Met Val Ser Thr Thr Leu His Val
   260 265 270
   Asp Ser Arg Met Ile Glu Asp Ala Ile Arg Ser His Ser Glu Ser Ala
   275 280 285
   Ser Pro Ser Ala Leu Ser Ser Ser Pro Asn Asn Leu Ser Pro Thr Gly
   290 295 300
   Trp Ser Gln Pro Lys Thr Pro Val Pro Ala Gln Arg Glu Arg Ala Pro
   305 310 315 320
   Gly Ser Gly Thr Gln Gln Lys Asn Lys Ile Arg Pro Arg Gly Gln Arg
   325 330 335
   Asp Ser Ser Tyr Tyr Trp Glu Ile Glu Ala Ser Glu Val Met Leu Ser
   340 345 350
   Thr Arg Ile Gly Ser Gly Ser Phe Gly Thr Val Tyr Lys Gly Lys Trp
   355 360 365
   His Gly Asp Val Ala Val Lys Ile Leu Lys Val Val Asp Pro Thr Pro
   370 375 380
   Glu Gln Leu Gln Ala Phe Arg Asn Glu Val Ala Val Leu Arg Lys Thr
   385 390 395 400
   Arg His Val Asn Ile Leu Leu Phe Met Gly Tyr Met Thr Lys Asp Asn
   405 410 415
   Leu Ala Ile Val Thr Gln Trp Cys Glu Gly Ser Ser Leu Tyr Lys His
   420 425 430
   Leu His Val Gln Glu Thr Lys Phe Gln Met Phe Gln Leu Ile Asp Ile
   435 440 445
   Ala Arg Gln Thr Ala Gln Gly Met Asp Tyr Leu His Ala Lys Asn Ile
   450 455 460
   Ile His Arg Asp Met Lys Ser Asn Asn Ile Phe Leu His Glu Gly Leu
   465 470 475 480
   Thr Val Lys Ile Gly Asp Phe Gly Leu Ala Thr Val Lys Ser Arg Trp
   485 490 495
   Ser Gly Ser Gln Gln Val Glu Gln Pro Thr Gly Ser Val Leu Trp Met
   500 505 510
   Ala Pro Glu Val Val Arg Met Gln Asp Asp Asn Pro Phe Ser Phe Gln
   515 520 525
   Ser Asp Val Tyr Ser Tyr Gly Ile Val Leu Tyr Glu Leu Met Ala Gly
   530 535 540
   Glu Leu Pro Tyr Ala His Ile Asn Asn Arg Asp Gln Ile Ile Phe Met
   545 550 555 560
   Val Gly Arg Gly Tyr Ala Ser Pro Asp Leu Ser Arg Leu Tyr Lys Asn
   565 570 575
   Cys Pro Lys Ala Met Lys Arg Leu Val Ala Asp Cys Val Lys Lys Val
   580 585 590
   Lys Glu Glu Arg Pro Leu Phe Pro Gln Ile Leu Ser Ser Ile Glu Leu
   595 600 605
   Leu Gln His Ser Leu Pro Lys Ile Asn Arg Ser Ala Ser Glu Pro Ser
   610 615 620
   Leu His Arg Ala Ala His Thr Glu Asp Ile Asn Ala Cys Thr Leu Thr
   625 630 635 640
   Thr Ser Pro Arg Leu Pro Val Phe
   645
(2) INFORMATION FOR SEQ ID NO:3:
   (i) SEQUENCE CHARACTERISTICS:
      (A) LENGTH: 648 amino acids
      (B) TYPE: amino acid
      (D) TOPOLOGY: linear
   (xi) SEQUENCE DESCRIPTION: SEQ ID NO:3:
   Met Glu His Ile Gln Gly Ala Trp Lys Thr Ile Ser Asn Gly Phe Gly
   1 5 10 15
   Leu Lys Asp Ala Val Phe Asp Gly Ser Ser Cys Ile Ser Pro Thr Ile
   20 25 30
   Val Gln Gln Phe Gly Tyr Gln Arg Arg Ala Ser Asp Asp Gly Lys Leu
   35 40 45
   Thr Asp Ser Ser Lys Thr Ser Asn Thr Ile Arg Val Phe Leu Pro Asn
       50                  55                  60
   Lys Gln Arg Thr Val Val Asn Val Arg Asn Gly Met Ser Leu His Asp
   65                  70                  75                  80
   Cys Leu Met Lys Ala Leu Lys Val Arg Gly Leu Gln Pro Glu Cys Cys
                   85                  90                  95
   Ala Val Phe Arg Leu Leu Gln Glu His Lys Gly Lys Lys Ala Arg Leu
               100                 105                 110
   Asp Trp Asn Thr Asp Ala Ala Ser Leu Ile Gly Glu Glu Leu Gln Val
           115                 120                 125
   Asp Phe Leu Asp His Val Pro Ile Thr Thr His Asn Phe Ala Arg Lys
       130                 135                 140
   Thr Phe Leu Lys Leu Ala Phe Cys Asp Ile Cys Gln Lys Phe Leu Leu
   145                 150                 155                 160
   Asn Gly Phe Arg Cys Gln Thr Cys Gly Tyr Lys Phe His Glu His Cys
                   165                 170                 175
   Ser Thr Lys Val Pro Thr Met Cys Val Asp Trp Ser Asn Ile Arg Gln
               180                 185                 190
   Leu Leu Leu Phe Pro Asn Ser Thr Val Gly Asp Ser Gly Val Pro Ala
           195                 200                 205
   Pro Pro Ser Phe Pro Met Arg Arg Met Arg Glu Ser Val Ser Arg Met
       210                 215                 220
   Pro Ala Ser Ser Gln His Arg Tyr Ser Thr Pro His Ala Phe Thr Phe
   225                 230                 235                 240
   Asn Thr Ser Ser Pro Ser Ser Glu Gly Ser Leu Ser Gln Arg Gln Arg
                   245                 250                 255
   Ser Thr Ser Thr Pro Asn Val His Met Val Ser Thr Thr Leu His Val
               260                 265                 270
   Asp Ser Arg Met Ile Glu Asp Ala Ile Arg Ser His Ser Glu Ser Ala
           275                 280                 285
   Ser Pro Ser Ala Leu Ser Ser Ser Pro Asn Asn Leu Ser Pro Thr Gly
       290                 295                 300
   Trp Ser Gln Pro Lys Thr Pro Val Pro Ala Gln Arg Glu Arg Ala Pro
   305                 310                 315                 320
   Gly Ser Gly Thr Gln Gln Lys Asn Lys Ile Arg Pro Arg Gly Gln Arg
                   325                 330                 335
   Asp Ser Ser Tyr Tyr Trp Glu Ile Glu Ala Ser Glu Val Met Leu Ser
   340 345 350
   Thr Arg Ile Gly Ser Gly Ser Phe Gly Thr Val Tyr Lys Gly Lys Trp
   355 360 365
   His Gly Asp Val Ala Val Lys Ile Leu Lys Val Val Asp Pro Thr Pro
   370 375 380
   Glu Gln Leu Gln Ala Phe Arg Asn Glu Val Ala Val Leu Arg Lys Thr
   385 390 395 400
   Arg His Val Asn Ile Leu Leu Phe Met Gly Tyr Met Thr Lys Asp Asn
   405 410 415
   Leu Ala Ile Val Thr Gln Trp Cys Glu Gly Ser Ser Leu Tyr Lys His
   420 425 430
   Leu His Val Gln Glu Thr Lys Phe Gln Met Phe Gln Leu Ile Asp Ile
   435 440 445
   Ala Arg Gln Thr Ala Gln Gly Met Asp Tyr Leu His Ala Lys Asn Ile
   450 455 460
   Ile His Arg Asp Met Lys Ser Asn Asn Ile Phe Leu His Glu Gly Leu
   465 470 475 480
   Thr Val Lys Ile Gly Asp Phe Gly Leu Ala Thr Val Lys Ser Arg Trp
   485 490 495
   Ser Gly Ser Gln Gln Val Glu Gln Pro Thr Gly Ser Val Leu Trp Met
   500 505 510
   Ala Pro Glu Val Ile Arg Met Gln Asp Asn Asn Pro Phe Ser Phe Gln
   515 520 525
   Ser Asp Val Tyr Ser Tyr Gly Ile Val Leu Tyr Glu Leu Met Ala Gly
   530 535 540
   Glu Leu Pro Tyr Ala His Ile Asn Asn Arg Asp Gln Ile Ile Phe Met
   545 550 555 560
   Val Gly Arg Gly Tyr Ala Ser Pro Asp Leu Ser Arg Leu Tyr Lys Asn
   565 570 575
   Cys Pro Lys Ala Met Lys Arg Leu Val Ala Asp Cys Val Lys Lys Val
   580 585 590
   Lys Glu Glu Arg Pro Leu Phe Pro Gln Ile Leu Ser Ser Ile Glu Leu
   595 600 605
   Leu Gln His Ser Leu Pro Lys Ile Asn Arg Ser Ala Ser Glu Pro Ser
   610 615 620
   Leu His Arg Ala Ala His Thr Glu Asp Ile Asn Ala Cys Thr Leu Thr
   625 630 635 640
   Thr Ser Pro Arg Leu Pro Val Phe
   645
(2) INFORMATION FOR SEQ ID NO:4:
   (i) SEQUENCE CHARACTERISTICS:
      (A) LENGTH: 648 amino acids
      (B) TYPE: amino acid
      (D) TOPOLOGY: linear
   (xi) SEQUENCE DESCRIPTION: SEQ ID NO:4:
   Met Glu His Ile Gln Gly Ala Trp Lys Thr Ile Ser Asn Gly Phe Gly
   1               5                   10                  15
   Leu Lys Asp Ala Val Phe Asp Gly Ser Ser Cys Ile Ser Pro Thr Ile
               20                  25                  30
   Val Gln Gln Phe Gly Tyr Gln Arg Arg Ala Ser Asp Asp Gly Lys Leu
           35                  40                  45
   Thr Asp Ser Ser Lys Thr Ser Asn Thr Ile Arg Val Phe Leu Pro Asn
       50                  55                  60
   Lys Gln Arg Thr Val Val Asn Val Arg Asn Gly Met Ser Leu His Asp
   65                  70                  75                  80
   Cys Leu Met Lys Ala Leu Lys Val Arg Gly Leu Gln Pro Glu Cys Cys
                   85                  90                  95
   Ala Val Phe Arg Leu Leu Gln Glu His Lys Gly Lys Lys Ala Arg Leu
               100                 105                 110
   Asp Trp Asn Thr Asp Ala Ala Ser Leu Ile Gly Glu Glu Leu Gln Val
           115                 120                 125
   Asp Phe Leu Asp His Val Pro Ile Thr Thr His Asn Phe Ala Arg Lys
       130                 135                 140
   Thr Phe Leu Lys Leu Ala Phe Cys Asp Ile Cys Gln Lys Phe Leu Leu
   145                 150                 155                 160
   Asn Gly Phe Arg Cys Gln Thr Cys Gly Tyr Lys Phe His Glu His Cys
                   165                 170                 175
   Ser Thr Lys Val Pro Thr Met Cys Val Asp Trp Ser Asn Ile Arg Gln
               180                 185                 190
   Leu Leu Leu Phe Pro Asn Ser Thr Val Gly Asp Ser Gly Val Pro Ala
           195                 200                 205
   Pro Pro Ser Phe Pro Met Arg Arg Met Arg Glu Ser Val Ser Arg Met
   210 215 220
   Pro Ala Ser Ser Gln His Arg Tyr Ser Thr Pro His Ala Phe Thr Phe
   225 230 235 240
   Asn Thr Ser Ser Pro Ser Ser Glu Gly Ser Leu Ser Gln Arg Gln Arg
   245 250 255
   Ser Thr Ser Thr Pro Asn Val His Met Val Ser Thr Thr Leu His Val
   260 265 270
   Asp Ser Arg Met Ile Glu Asp Ala Ile Arg Ser His Ser Glu Ser Ala
   275 280 285
   Ser Pro Ser Ala Leu Ser Ser Ser Pro Asn Asn Leu Ser Pro Thr Gly
   290 295 300
   Trp Ser Gln Pro Lys Thr Pro Val Pro Ala Gln Arg Glu Arg Ala Pro
   305 310 315 320
   Gly Ser Gly Thr Gln Gln Lys Asn Lys Ile Arg Pro Arg Gly Gln Arg
   325 330 335
   Asp Ser Ser Tyr Tyr Trp Glu Ile Glu Ala Ser Glu Val Met Leu Ser
   340 345 350
   Thr Arg Ile Gly Ser Gly Ser Phe Gly Thr Val Tyr Lys Gly Lys Trp
   355 360 365
   His Gly Asp Val Ala Val Lys Ile Leu Lys Val Val Asp Pro Thr Pro
   370 375 380
   Glu Gln Leu Gln Ala Phe Arg Asn Glu Val Ala Val Leu Arg Lys Thr
   385 390 395 400
   Arg His Val Asn Ile Leu Leu Phe Met Gly Tyr Met Thr Lys Asp Asn
   405 410 415
   Leu Ala Ile Val Thr Gln Trp Cys Glu Gly Ser Ser Leu Tyr Lys His
   420 425 430
   Leu His Val Gln Glu Thr Lys Phe Gln Met Phe Gln Leu Ile Asp Ile
   435 440 445
   Ala Arg Gln Thr Ala Gln Gly Met Asp Tyr Leu His Ala Lys Asn Ile
   450 455 460
   Ile His Arg Asp Met Lys Ser Asn Asn Ile Phe Leu His Glu Gly Leu
   465 470 475 480
   Thr Val Lys Ile Gly Asp Phe Gly Leu Ala Thr Val Lys Ser Arg Trp
   485 490 495
   Ser Gly Ser Gln Gln Val Glu Gln Pro Thr Gly Ser Val Leu Trp Met
   500 505 510
   Ala Pro Glu Val Ile Arg Met Gln Asp Asp Asn Pro Phe Ser Ser Gln
   515 520 525
   Ser Asp Val Tyr Ser Tyr Gly Ile Val Leu Tyr Glu Leu Met Ala Gly
   530 535 540
   Glu Leu Pro Tyr Ala His Ile Asn Asn Arg Asp Gln Ile Ile Phe Met
   545 550 555 560
   Val Gly Arg Gly Tyr Ala Ser Pro Asp Leu Ser Arg Leu Tyr Lys Asn
   565 570 575
   Cys Pro Lys Ala Met Lys Arg Leu Val Ala Asp Cys Val Lys Lys Val
   580 585 590
   Lys Glu Glu Arg Pro Leu Phe Pro Gln Ile Leu Ser Ser Ile Glu Leu
   595 600 605
   Leu Gln His Ser Leu Pro Lys Ile Asn Arg Ser Ala Ser Glu Pro Ser
   610 615 620
   Leu His Arg Ala Ala His Thr Glu Asp Ile Asn Ala Cys Thr Leu Thr
   625 630 635 640
   Thr Ser Pro Arg Leu Pro Val Phe
   645
(2) INFORMATION FOR SEQ ID NO:5:
   (i) SEQUENCE CHARACTERISTICS:
      (A) LENGTH: 648 amino acids
      (B) TYPE: amino acid
      (D) TOPOLOGY: linear
   (xi) SEQUENCE DESCRIPTION: SEQ ID NO:5:
   Met Glu His Ile Gln Gly Ala Trp Lys Thr Ile Ser Asn Gly Phe Gly
   1 5 10 15
   Leu Lys Asp Ala Val Phe Asp Gly Ser Ser Cys Ile Ser Pro Thr Ile
   20 25 30
   Val Gln Gln Phe Gly Tyr Gln Arg Arg Ala Ser Asp Asp Gly Lys Leu
   35 40 45
   Thr Asp Ser Ser Lys Thr Ser Asn Thr Ile Arg Val Phe Leu Pro Asn
   50 55 60
   Lys Gln Arg Thr Val Val Asn Val Arg Asn Gly Met Ser Leu His Asp
   65 70 75 80
   Cys Leu Met Lys Ala Leu Lys Val Arg Gly Leu Gln Pro Glu Cys Cys
   85 90 95
   Ala Val Phe Arg Leu Leu Gln Glu His Lys Gly Lys Lys Ala Arg Leu
   100 105 110
   Asp Trp Asn Thr Asp Ala Ala Ser Leu Ile Gly Glu Glu Leu Gln Val
   115 120 125
   Asp Phe Leu Asp His Val Pro Ile Thr Thr His Asn Phe Ala Arg Lys
   130 135 140
   Thr Phe Leu Lys Leu Ala Phe Cys Asp Ile Cys Gln Lys Phe Leu Leu
   145 150 155 160
   Asn Gly Phe Arg Cys Gln Thr Cys Gly Tyr Lys Phe His Glu His Cys
   165 170 175
   Ser Thr Lys Val Pro Thr Met Cys Val Asp Trp Ser Asn Ile Arg Gln
   180 185 190
   Leu Leu Leu Phe Pro Asn Ser Thr Val Gly Asp Ser Gly Val Pro Ala
   195 200 205
   Pro Pro Ser Phe Pro Met Arg Arg Met Arg Glu Ser Val Ser Arg Met
   210 215 220
   Pro Ala Ser Ser Gln His Arg Tyr Ser Thr Pro His Ala Phe Thr Phe
   225 230 235 240
   Asn Thr Ser Ser Pro Ser Ser Glu Gly Ser Leu Ser Gln Arg Gln Arg
   245 250 255
   Ser Thr Ser Thr Pro Asn Val His Met Val Ser Thr Thr Leu His Val
   260 265 270
   Asp Ser Arg Met Ile Glu Asp Ala Ile Arg Ser His Ser Glu Ser Ala
   275 280 285
   Ser Pro Ser Ala Leu Ser Ser Ser Pro Asn Asn Leu Ser Pro Thr Gly
   290 295 300
   Trp Ser Gln Pro Lys Thr Pro Val Pro Ala Gln Arg Glu Arg Ala Pro
   305 310 315 320
   Gly Ser Gly Thr Gln Gln Lys Asn Lys Ile Arg Pro Arg Gly Gln Arg
   325 330 335
   Asp Ser Ser Tyr Tyr Trp Glu Ile Glu Ala Ser Glu Val Met Leu Ser
   340 345 350
   Thr Arg Ile Gly Ser Gly Ser Phe Gly Thr Val Tyr Lys Gly Lys Trp
   355 360 365
   His Gly Asp Val Ala Val Lys Ile Leu Lys Val Val Asp Pro Thr Pro
   370 375 380
   Glu Gln Leu Gln Ala Phe Arg Asn Glu Val Ala Val Leu Arg Lys Thr
   385 390 395 400
   Arg His Val Asn Ile Leu Leu Phe Met Gly Tyr Met Thr Lys Asp Asn
   405 410 415
   Leu Ala Ile Val Thr Gln Trp Cys Glu Gly Ser Ser Leu Tyr Lys His
   420 425 430
   Leu His Val Gln Glu Thr Lys Phe Gln Met Phe Gln Leu Ile Asp Ile
   435 440 445
   Ala Arg Gln Thr Ala Gln Gly Met Asp Tyr Leu His Ala Lys Asn Ile
   450 455 460
   Ile His Arg Asp Met Lys Ser Asn Asn Ile Phe Leu His Glu Gly Leu
   465 470 475 480
   Thr Val Lys Ile Gly Asp Phe Gly Leu Ala Thr Val Lys Ser Arg Trp
   485 490 495
   Ser Gly Ser Gln Gln Val Glu Gln Pro Thr Gly Ser Val Leu Trp Met
   500 505 510
   Ala Pro Glu Val Ile Arg Met Gln Asp Asp Asn Pro Phe Ser Phe Gln
   515 520 525
   Ser Thr Cys Thr Phe Tyr Gly Ile Val Leu Tyr Glu Leu Met Ala Gly
   530 535 540
   Glu Leu Pro Tyr Ala His Ile Asn Asn Arg Asp Gln Ile Ile Phe Met
   545 550 555 560
   Val Gly Arg Gly Tyr Ala Ser Pro Asp Leu Ser Arg Leu Tyr Lys Asn
   565 570 575
   Cys Pro Lys Ala Met Lys Arg Leu Val Ala Asp Cys Val Lys Lys Val
   580 585 590
   Lys Glu Glu Arg Pro Leu Phe Pro Gln Ile Leu Ser Ser Ile Glu Leu
   595 600 605
   Leu Gln His Ser Leu Pro Lys Ile Asn Arg Ser Ala Ser Glu Pro Ser
   610 615 620
   Leu His Arg Ala Ala His Thr Glu Asp Ile Asn Ala Cys Thr Leu Thr
   625 630 635 640
   Thr Ser Pro Arg Leu Pro Val Phe
   645
(2) INFORMATION FOR SEQ ID NO:6:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:6:
   AACTTGTGGT GGTTGGACCT 20
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:7:
   TCATCCACAA AGTGATTCTG 20
(2) INFORMATION FOR SEQ ID NO:8:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:8:
   AGGAGACCAA GTTTCAGATG 20
(2) INFORMATION FOR SEQ ID NO:9:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:9:
   GCGTGCAAGC ATTGATATCC 20
(2) INFORMATION FOR SEQ ID NO:10:
   (i) SEQUENCE CHARACTERISTICS:
      (A) LENGTH: 1947 base pairs
      (B) TYPE: nucleic acid
      (C) STRANDEDNESS: double
      (D) TOPOLOGY: linear
   (xi) SEQUENCE DESCRIPTION: SEQ ID NO:10:
   ATGGAGCACA TACAGGGAGC TTGGAAGACG ATCAGCAATG GCTTTGGACT CAAAGATGCG   60
   GTGTTTGATG GCTCCAGCTG CATCTCCCCT ACCATTGTTC AGCAGTTTGG CTATCAGCGC  120
   CGGGCCTCAG ATGATGGCAA GCTCACGGAT TCTTCTAAGA CAAGCAATAC TATCCGGGTT  180
   TTCTTGCCGA ATAAGCAAAG GACTGTGGTC AATGTGCGGA ATGGAATGAG CTTACATGAC  240
   TGCCTTATGA AAGCTCTGAA GGTGAGAGGC CTGCAGCCAG AGTGCTGTGC AGTGTTCAGA  300
   CTTCTCCAGG AACACAAAGG TAAGAAAGCA CGCTTAGATT GGAACACCGA TGCCGCCTCT  360
   CTGATTGGAG AAGAACTGCA AGTGGATTTT TTGGATCATG TTCCCATCAC AACTCACAAC  420
   TTTGCTCGGA AAACGTTCCT GAAGCTTGCA TTCTGTGACA TCTGTCAGAA GTTCCTGCTA  480
   AATGGATTTC GATGTCAGAC TTGTGGCTAC AAGTTTCATG AGCACTGTAG CACCAAAGTA  540
   CCTACTATGT GTGTGGACTG GAGTAATATC AGACAGCTCT TGCTGTTTCC AAATTCCACT  600
   GTTGGTGACA GTGGAGTCCC AGCACCACCT TCTTTCCCAA TGCGTCGGAT GCGAGAATCT  660
   GTTTCCCGGA TGCCTGCTAG TTCCCAGCAC AGATACTCTA CACCCCATGC CTTCACTTTC  720
   AACACCTCCA GCCCTTCCTC AGAAGGTTCC CTCTCCCAGA GGCAGAGGTC AACGTCCACT  780
   CCCAATGTCC ACATGGTCAG CACCACCCTG CATGTGGACA GCAGGATGAT TGAGGATGCA  840
   ATTCGAAGTC ACAGTGAATC AGCCTCACCT TCAGCCCTGT CCAGCAGCCC AAACAACCTG  900
   GGTCCAACAG GCTGGTCACA GCCCAAAACC CCCGTGCCAG CACAAAGAGA GCGGGCACCA  960
   GGATCTGGGA CCCAGCAAAA AAACAAAATT AGGCCTCGTG GGCAGAGAGA CTCGAGTTAT 1020
   TACTGGGAAA TAGAAGCCAG TGAGGTGATG CTGTCTACTC GGATCGGGTC AGGTTCCTTT 1080
   GGCACTGTGT ACAAGGGCAA GTGGCATGGA GATGTTGCAG TAAAGATCCT AAAGGTGGTT 1140
   GACCCAACTC CAGAGCAACT TCAGGCCTTC AGGAACGAGG TGGCTGTTTT GCGCAAAACA 1200
   CGGCATGTTA ACATCCTGCT GTTCATGGGG TACATGACAA AGGACAACCT GGCGATTGTG 1260
   ACTCAGTGGT GTGAAGGCAG CAGTCTCTAC AAACACCTGC ATGTCCAGGA GACCAAATTC 1320
   CAGATGTTCC AGCTAATTGA CATTGCCCGA CAGACAGCTC AGGGAATGGA CTATTTGCAT 1380
   GCAAAGAACA TCATCCACAG AGACATGAAA TCCAACAATA TATTTCTCCA TGAAGGCCTC 1440
   ACGGTGAAAA TTGGAGATTT TGGTTTGGCA ACAGTGAAGT CACGCTGGAG TGGTTCTCAG 1500
   CAGGTTGAAC AGCCCACTGG CTCTGTGCTG TGGATGGCCC CAGAAGTAAT CCGGATGCAG 1560
   GATGACAACC CGTTCAGCTT CCAGTCCGAC GTGTACTCGT ACGGCATCGT GCTGTACGAG 1620
   CTGATGGCTG GGGAGCTTCC CTACGCCCAC ATCAACAACC GAGACCAGAT CATCTTCATG 1680
   GTAGGCCGTG GGTATGCATC CCCTGATCTC AGCAGGCTCT ACAAGAACTG CCCCAAGGCA 1740
   ATGAAGAGGT TGGTGGCTGA CTGTGTGAAG AAAGTCAAAG AAGAGAGACC TTTGTTTCCC 1800
   CAGATCCTGT CTTCCATCGA GCTGCTTCAG CACTCTCTGC CGAAAATCAA CAGGAGCGCC 1860
   TCTGAGCCTT CCCTGCATCG GGCAGCTCAC ACTGAGGACA TCAATGCTTG CACGCTGACT 1920
   ACATCCCCAA GGCTACCAGT CTTCTAG                                    1947
(2) INFORMATION FOR SEQ ID NO:11:
   (i) SEQUENCE CHARACTERISTICS:
      (A) LENGTH: 1947 base pairs
      (B) TYPE: nucleic acid
      (C) STRANDEDNESS: double
      (D) TOPOLOGY: linear
   (ix) FEATURE:
      (A) NAME/KEY: CDS
      (B) LOCATION: 1..1944
   (xi) SEQUENCE DESCRIPTION: SEQ ID NO:11:
   ATG GAG CAC ATA CAG GGA GCT TGG AAG ACG ATC AGC AAT GGT TTT GGA   48
   TTC AAA GAT GCC GTG TTT GAT GGC TCC AGC TGC ATC TCT CCT ACA ATA   96
   GTT CAG CAG TTT GGC TAT CAG CGC CGG GCA TCA GAT GAT GGC AAA CTC  144
   ACA GAT CCT TCT AAG ACA AGC AAC ACT ATC CGT GTT TTC TTG CCG AAC  192
   AAG CAA AGA ACA GTG GTC AAT GTG CGA AAT GGA ATG AGC TTG CAT GAC  240
   TGC CTT ATG AAA GCA CTC AAG GTG AGG GGC CTG CAA CCA GAG TGC TGT  288
   GCA GTG TTC AGA CTT CTC CAC GAA CAC AAA GGT AAA AAA GCA CGC TTA  336
   GAT TGG AAT ACT GAT GCT GCG TCT TTG ATT GGA GAA GAA CTT CAA GTA  384
   GAT TTC CTG GAT CAT GTT CCC CTC ACA ACA CAC AAC TTT GCT CGG AAG  432
   ACG TTC CTG AAG CTT GCC TTC TGT GAC ATC TGT CAG AAA TTC CTG CTC  480
   AAT GGA TTT CGA TGT CAG ACT TGT GGC TAC AAA TTT CAT GAG CAC TGT  528
   AGC ACC AAA GTA CCT ACT ATG TGT GTG GAC TGG AGT AAC ATC AGA CAA  576
   CTC TTA TTG TTT CCA AAT TCC ACT ATT GGT GAT AGT GGA GTC CCA GCA  624
   CTA CCT TCT TTG ACT ATG CGT CGT ATG CGA GAG TCT GTT TCC AGG ATG  672
   CCT GTT AGT TCT CAG CAC AGA TAT TCT ACA CCT CAC GCC TTC ACC TTT  720
   AAC ACC TCC AGT CCC TCA TCT GAA GGT TCC CTC TCC CAG AGG CAG AGG  768
   TCG ACA TCC ACA CCT AAT GTC CAC ATG GTC AGC ACC ACG CTG CCT GTG  816
   GAC AGC AGG ATG ATT GAG GAT GCA ATT CGA AGT CAC AGC GAA TCA GCC  864
   TCA CCT TCA GCC CTG TCC AGT AGC CCC AAC AAT CTG AGC CCA ACA GGC  912
   TGG TCA CAG CCG AAA ACC CCC GTG CCA GCA CAA AGA GAG CGG GCA CCA  960
   GTA TCT GGG ACC CAG GAG AAA AAC AAA ATT AGG CCT CGT GGA CAG AGA 1008
   GAT TCA AGC TAT TAT TGG GAA ATA GAA GCC AGT GAA GTG ATG CTG TCC 1056
   ACT CGG ATT GGG TCA GGC TCT TTT GGA ACT GTT TAT AAG GGT AAA TGG 1104
   CAC GGA GAT GTT GCA GTA AAG ATC CTA AAG GTT GTC GAC CCA ACC CCA 1152
   GAG CAA TTC CAG GCC TTC AGG AAT GAG GTG GCT GTT CTG CGC AAA ACA 1200
   CGG CAT GTG AAC ATT CTG CTT TTC ATG GGG TAC ATG ACA AAG GAC AAC 1248
   CTG GCA ATT GTG ACC CAG TGG TGC GAG GGC AGC AGC CTC TAC AAA CAC 1296
   CTG CAT GTC CAG GAG ACC AAG TTT CAG ATG TTC CAG CTA ATT GAC ATT 1344
   GCC CGG CAG ACG GCT CAG GGA ATG GAC TAT TTG CAT GCA AAG AAC ATC 1392
   ATC CAT AGA GAC ATG AAA TCC AAC AAT ATA TTT CTC CAT GAA GGC TTA 1440
   ACA GTG AAA ATT GGA GAT TTT GGT TTG GCA ACA GTA AAG TCA CGC TGG 1488
   AGT GGT TCT CAG CAG GTT GAA CAA CCT ACT GGC TCT GTC CTC TGG ATG 1536
   GCC CCA GAG GTG ATC CGA ATG CAG GAT AAC AAC CCA TTC AGT TTC CAG 1584
   TCG GAT GTC TAC TCC TAT GGC ATC GTA TTG TAT GAA CTG ATG ACG GGG 1632
   GAG CTT CCT TAT TCT CAC ATC AAC AAC CGA GAT CAG ATC ATC TTC ATG 1680
   GTG GGC CGA GGA TAT GCC TCC CCA GAT CTT AGT AAG CTA TAT AAG AAC 1728
   TGC CCC AAA GCA ATG AAG AGG CTG GTA GCT GAC TGT GTG AAG AAA GTA 1776
   AAG GAA GAG AGG CCT CTT TTT CCC CAG ATC CTG TCT TCC ATT GAG CTG 1824
   CTC CAA CAC TCT CTA CCG AAG ATC AAC CGG AGC GCT TCC GAG CCA TCC 1872
   TTG CAT CGG GCA GCC CAC ACT GAG GAT ATC AAT GCT TGC ACG CTG ACC 1920
   ACG TCC CCG AGG CTG CCT GTC TTC TAG                             1947
(2) INFORMATION FOR SEQ ID NO:12:
   (i) SEQUENCE CHARACTERISTICS:
      (A) LENGTH: 648 amino acids
      (B) TYPE: amino acid
      (D) TOPOLOGY: linear
   (ii) MOLECULE TYPE: protein
   (xi) SEQUENCE DESCRIPTION: SEQ ID NO:12:

## Description

### Field of the Invention

The present invention relates to (1) a method of identifying an individual at an increased risk for developing cancer, (2) a method for determining a prognosis of patients afflicted with cancer, and (3) a method for determining the proper course of treatment for a patient afflicted with cancer.

### Background Information

Lung cancer claims more lives in the United States than any other neoplasm (R.S. Finley, Am. Pharm. NS29, 39 (1989)), and of the various forms lung adenocarcinomas have one of the worst prognoses (T.P. Miller, Semin. Oncol. 17, 11 (1990)). The incidence of adenocarcinoma of the lung (ACL) in the United States is also quickly rising (I. Linnoila, Hematol. Oncol. North. Am. 4, 1027 (1990); J.B. Sorensen, H.H. Hansen, Cancer Surviv. 8, 671 (1989)). In order to gain insight into this complex and deadly disease, a model system for its study has been developed. For such a model to provide clinically relevant data several criteria must be met. The tumors produced should be histologically equivalent to their human counterparts, tumor induction must be reliable and reproducible, and the numbers generated must be great enough to provide statistical significance. To satisfy these conditions a system has been created which uses two inbred mouse strains (NFS/n and AKR) along with transplacental exposure to the potent carcinogen N-ethyl-N-nitrosourea (ENU) and promotion with the antioxidant butylated hydroxytoluene (BHT). The resulting tumors were examined for altered expression or structural mutations of genes implicated in lung tumor development such as ras myc, and raf oncogenes (C.D. Little et al., Nature 306, 194 (1983); P.E. Kiefer et al., Cancer Res., 47, 6236 (1987); E. Santos et al., Science 223, 661 (1984); S. Rodenhuis, N. Enal. J. Med. 317, 929 (1987); M. Barbacid, Eur. J. Clin. Invest. 20, 225 (1990); U.R. Rapp et al., J. Int. Assoc. for the Study of Lun Cancer 4, 162 (1988); M.J. Birrer et al., Ann. Rev. Med. 40, 305 (1989); G. Sithanandam et al., Oncogene 4, 451 (1989)).

raf proto-oncogenes are evolutionarily highly conserved genes encoding cytoplasmic serine/threonine specific kinases, which function in mitogen signal transduction (reviewed in U.R. Rapp et al., The Oncogene Handbook, T. Curran et al., Eds. (Elsevier Science Publishers, The Netherlands, 1988), pp. 115-154; U.R. Rapp, Oncogene 6, 495 (1991)). The three known active members in the raf family encode phosphoproteins of similar size (72/74 kD for Raf-1; 68 kD for A-Raf-1, and 74 kD for B-Raf (U.R. Rapp et al., in Retroviruses and Human Pathology, R. Gallo et al., Eds. (Humana Press, Clifton, New Jersey 1985), pp. 449-472; T.W. Beck et al., Nucleic Acids Res. 15, 595 (1987); G. Sithanandam et al., Oncogene 5, 1775 (1990))). Raf-1 was first identified as the cellular homologue of v-raf (H.W. Jansen et al., Nature 307, 218 (1984)), the transforming gene of 3611 MSV (U.R. Rapp et al., J. Virol. 45, 914 (1983); U.R. Rapp et al., Proc. Natl. Acad. Sci. USA 80, 4218 (1983)). Amino acid comparisons of raf family genes shows three conserved regions [CR1, CR2, CR3] (T.W. Beck et al., Nucleic Acids Res. 15, 595 (1987)); CR1 is a regulatory region surrounding a Cys finger consensus sequence, CR2 is a serine/threonine rich region, and CR3 represents the kinase domain. Raf-1 has been mapped to chromosome 3p25 in humans (S.J. O'Brien et al., Science 223, 71 (1984)), and this region has been found to be frequently altered in small cell lung carcinoma (SCLC) (J. Whang-Peng et al., Cancer Genet. Cytogenet. 6, 119 (1982); J.M. Ibson et al., J. Cell. Biochem. 33, 267 (1987)), familial renal cell carcinoma (A.J. Cohen et al., N. Enal. J. Med. 301, 592 (1979); G. Kovacs et al., Int. J. Cancer 40, 171 (1987)), mixed parotid gland tumors (J. Mark et al., Hereditas 96, 141 (1982)), and ovarian cancer (K. Tanaka et al., Cancer Genet. Cytogenet. 43, 1 (1989)).

Raf genes are differentially expressed in various tissues (S.M. Storm et al., Oncogene 5, 345 (1990)). c-raf-1 has been found to be expressed ubiquitously, though absolute levels vary between tissues. A-raf-1 is present predominantly in the urogenital tissues, whereas B-Raf is most abundant in cerebrum and testis. The ubiquitous c-Raf-1 kinase is regulated by tyrosine and serine phosphorylations that result from activated growth factor receptor kinases (D.K. Morrison et al., Cell 58, 648 (1989); D.K. Morrison et al., Proc. Natl. Acad. Sci. USA 85, 8855 (1989); K.S. Kovacina et al., J. Biol. Chem. 265, 12115 (1990); P.J. Blackshear et al., J. Biol. Chem. 265, 12131 (1990); M.P. Carroll et al., J. Biol. Chem. 265, 19812 (1990); J.N. Siegel et al., J. Biol. Chem. 265, 18472 (1990); B.C. Turner et al., Proc. Natl. Acad. Sci. USA 88, 1227 (1991); M. Baccarini et al., EMBO J. 9, 3649 (1990); H. App et al., Mol. Cell. Biol. 11, 913 (1991)). Raf-1 operates downstream of Ras in mitogen signal transduction as judged by experiments using antibody microinjection (M.R. Smith et al., Nature 320, 540 (1986)), c-raf-1 antisense expression constructs (W. Kolch et al., Nature 349, 426 (1991)), dominant negative mutants (W. Kolch et al., Nature 349, 426 (1991)), and Raf revertant cells. Studies with NIH3T3 cells and brain tissue demonstrated that mitogen treatment induces Raf-1 kinase activity and causes a transitory relocation of the active enzyme from the cytoplasm to the nucleus and perinuclear area (Z. Olàh et al., Exp. Brain. Res. (in press); U.R. Rapp et al., in Cold Spring Harbor Symposia on Quantitative Biology, Vol. LIII, Eds. (Cold Spring Harbor Laboratory Press, Cold Spring Harbor, NY 1988) pp. 173-184).

Raf-1 coupling has been examined in more than a dozen receptor systems and all strong mitogens stimulated Raf-1 kinase activity (U.R. Rapp, Oncogene 6, 495 (1991); D.K. Morrison et al., Cell 58, 648 (1989); D.K. Morrison et al., Proc. Natl. Acad. Sci. USA 85, 8855 (1989); K.S. Kovacina et al., J. Biol. Chem. 265, 12115 (1990); P.J. Blackshear et al., J. Biol. Chem. 265, 12131 (1990); M.P. Carroll et al., J. Biol. Chem. 265, 19812 (1990); J.N. Siegel et al., J. Biol. Chem. 265, 18472 (1990); B.C. Turner et al., Proc. Natl. Acad. Sci. USA 88, 1227 (1991); M. Baccarini et al., EMBO J. 9, 3649 (1990); H. App et al., Mol. Cell. Biol. 11, 913 (1991)), and this stimulation correlated with an increase in Raf-1 phosphorylation leading to a shift in apparent molecular weight.

### SUMMARY OF THE INVENTION

According to the present invention, the presence of a cancer in a subject is identified by detecting the presence of a point mutation in the c-raf-1 gene of the subject. The point mutation may be in a region encoding amino-acids 450 to 630, e.g. 500 to 550, and preferably 514 to 535, e.g. the base encoding amino-acid 533, of SEQ ID NO:12.

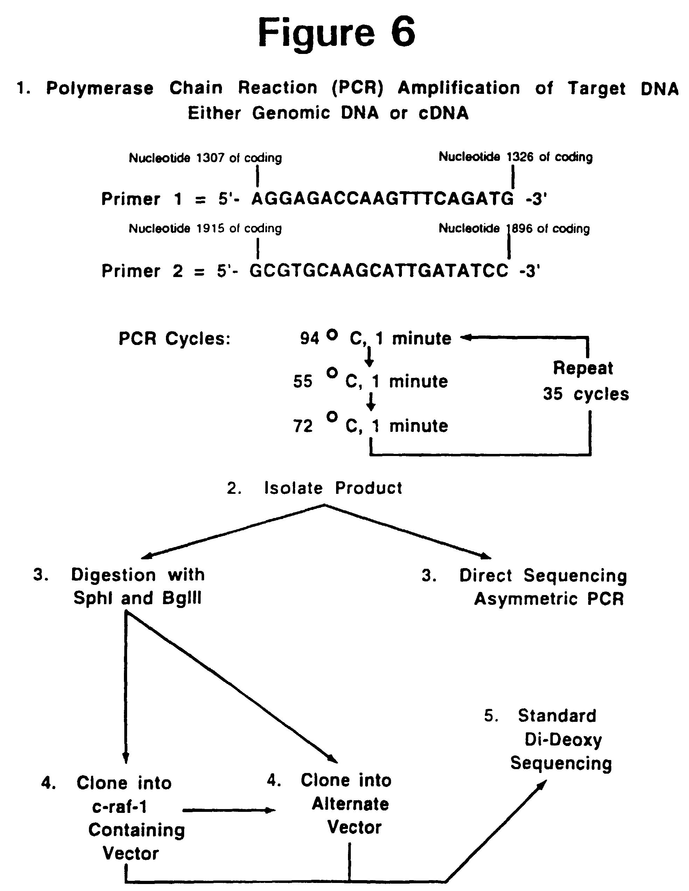

If desired, prior to detection, the gene or the given region may be amplified, e.g. by the polymerase chain reaction (PCR). Products of amplification may be analyzed for evidence of mutation, preferably by DNA sequencing. The PCR method preferably employs primers respectively comprising the sequences of SEQ ID NO: 8 and SEQ ID NO:9. The method may comprise the steps shown in Figure 6.

The present invention can be used to identify cancers such as lung cancer, T-cell lymphomas, renal cell carcinoma, and variant carcinoma and mixed parotid gland tumors. It is particularly suitable for identifying lung adenocarcinomas.

By means of the invention, patients having a mutation in the given region may be identified, and appropriate treatment prescribed where there is a reduced chance of survival or a reduced time to relapse. Administration of therapeutic agents (cytotoxic or cytostatic) tailored to recognize the mutant Raf-1 protein but not normal Raf-1 could specifically target tumor cells for death or growth inhibition. Such agents may comprise cytotoxic T-cells, antibodies, and/or specifically designed chemical compounds. By identifying patients lacking mutations in the given region, it is possible to provide treatment appropriate for patients having a greater chance of survival or being less likely to suffer disease relapse.

### BRIEF DESCRIPTION OF THE DRAWINGS

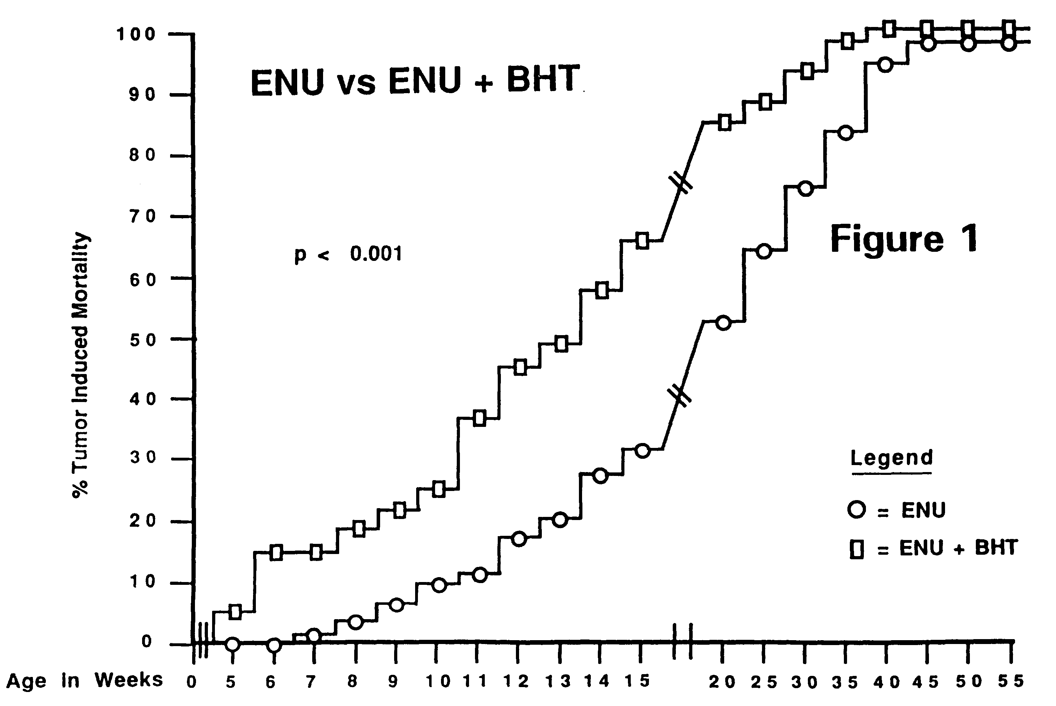

Figure 1. Effect of BHT promotion on ENU tumorigenesis in NFS/n x AKR mice. The Y-axis represents percent tumor induced mortality within each group, and the X-axis reflects age in weeks. All animals were exposed to ENU transplacentally at a dose of 0.5mM/Kg of mother's body weight on day 16 of gestation (presence of vaginal plug was scored as day one). At two weeks of age mice were weaned into two separate groups and separated by sex. Both groups were housed in identical cages and supplied with food (Purina Lab Chow) and water ad libitum. Beginning at three weeks of age, group 2A (O) was given weekly intraperitoneal (i.p.) injection of corn oil (0.1 ml), and group 2B (o) received weekly i.p. injections of BHT (20 mg/Kg of body weight) dissolved in corn oil. Administration of BHT reduces the mean age of mortality from approximately 20 weeks to 13, and decreases the initial age of mortality. These curves are significantly different (p≤ 0.001) as judged by a 2-tailed Cox test. In both groups the rate of tumorigenesis was identical for males and females.

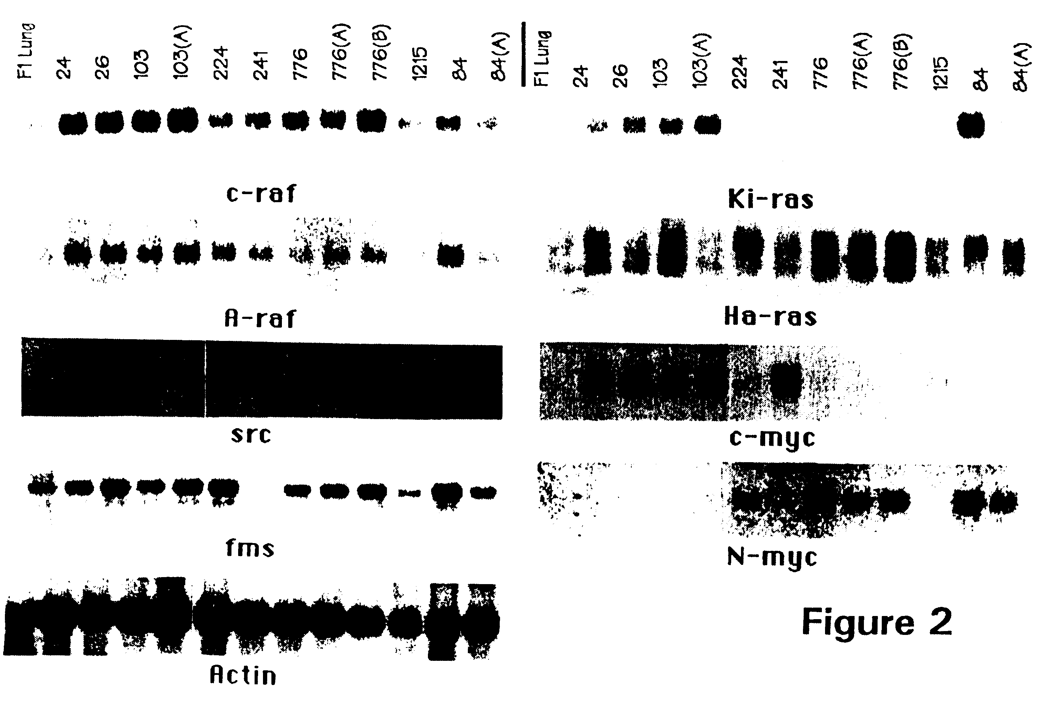

Figure 2. Northern blot analysis of proto-oncogene expression levels in ENU induced tumors.

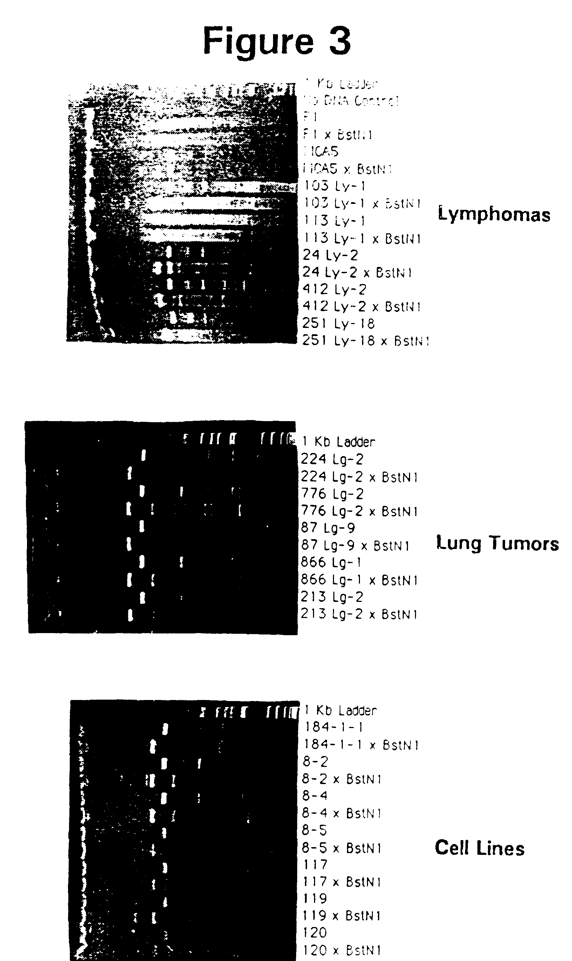

Figure 3. Diagnostic digestion of PCR amplified Ki-ras genes from ENU induced tumors. Genomic DNA was isolated from a cesium chloride gradient during RNA preparations. In each case 10 ng was amplified via PCR (95°C, 5 min. followed by 35 cycles of 95°C, 1 min. → 55°C, 1 min. → 72°, 1 min.) with 2 units of Taq I polymerase. The primers used (K1; 5'-AACTTGTGGTGGTTGGACCT-3'→ (SEQ ID NO:6) and K2; <= 3'-GTCTTAGTGAAACACCTACT-5' (SEQ ID NO:7)) generate a 79 b.p. product. The primer K1 ends at codon 12 and contains a mismatch from normal mouse (Ki-ras sequence its 18th nucleotide (G → C) creating a BstNI site (CCTGG) in conjunction with a normal codon 12 (GGT). Digestion of amplified product from a normal allele with BstNI produces two products of 19 and 60 b.p., whereas a mutation in one of the first two positions of codon 12 will eliminate the BstNI site. The presence of two normal alleles results in all of the product being cleaved and the presence of one mutant and one normal allele will result in only half of the product cut. In the three panels each sample was run in duplicate, uncut and cut with BstNI. F1 is DNA from an untreated NFS/n X AKR F1 mouse, and MCA5 is a murine cell line known to harbor a mutant K-ras codon 12 allele. One lymphoma (24Ly) and one cell line (117; derived from a lung adenocarcinoma) display a mutated Ki-ras codon 12 allele; however, 24Ly was a passaged tumor and examination of the original tumor showed two normal alleles indicating that this mutation was acquired during passaging.

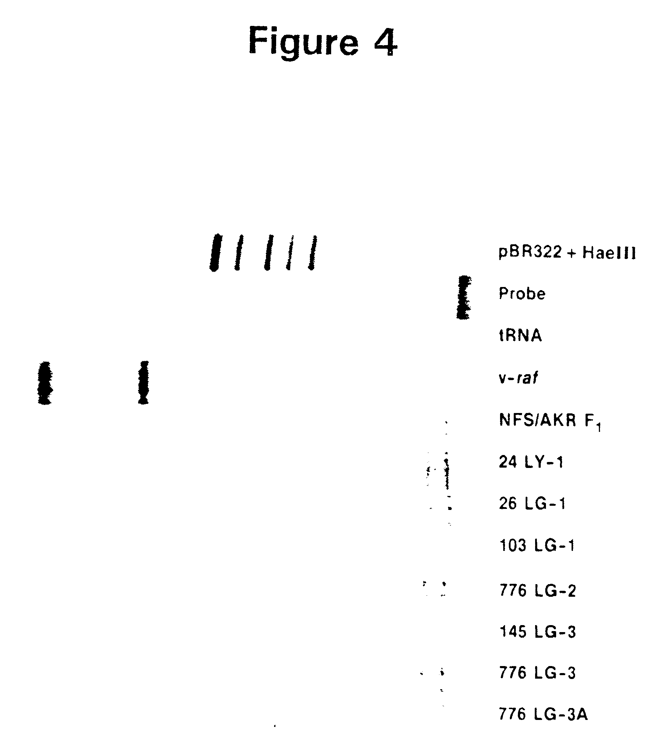

Figure 4. c-raf-1 RNAse protection analysis of ENU induced tumors. The probe used was a ³²P labeled antisense transcript from the 3' non-coding region of a mouse c-raf-1 cDNA to the 3' most StuI site. Hybridization of this probe with normal RNA results in a protected fragment of 1.2kb covering the region encoding the Raf-1 kinase domain. One µg of poly(A)+ RNA from each tumor and 5 µg of F1 RNA (in order to get comparable signals) was hybridized for 12 hours at 52°C with 200,000 cpm of ³²P labeled mouse c-raf antisense transcript. Hybrids were then digested for 30 minutes with 25 µg RNAse A and 33 units of RNAse T1 at room temperature. Digested hybrids were then incubated with 50 µg of proteinase K, phenol/chloroform extracted, ethanol precipitated, and resuspended in loading dye containing 80% formamide. Samples were then run on 6% polyacrylamide denaturing sequencing gels at 65 watts. Gels were vacuum dried at 80 degrees C and exposed to X-ray film. Probe is undigested probe alone; tRNA is probe hybridized to non-specific RNA; v-raf is probe hybridized to RNA from a v-raf transformed cell line and the bands detected represent single base mismatches between murine c-raf and v-raf; NFS/AKR F1 is probe hybridized with RNA from a normal (untreated) mouse; 24 LY is probe hybridized with RNA from a lymphoma; and the remaining lanes are probe hybridized with RNA isolated from lung tumors.

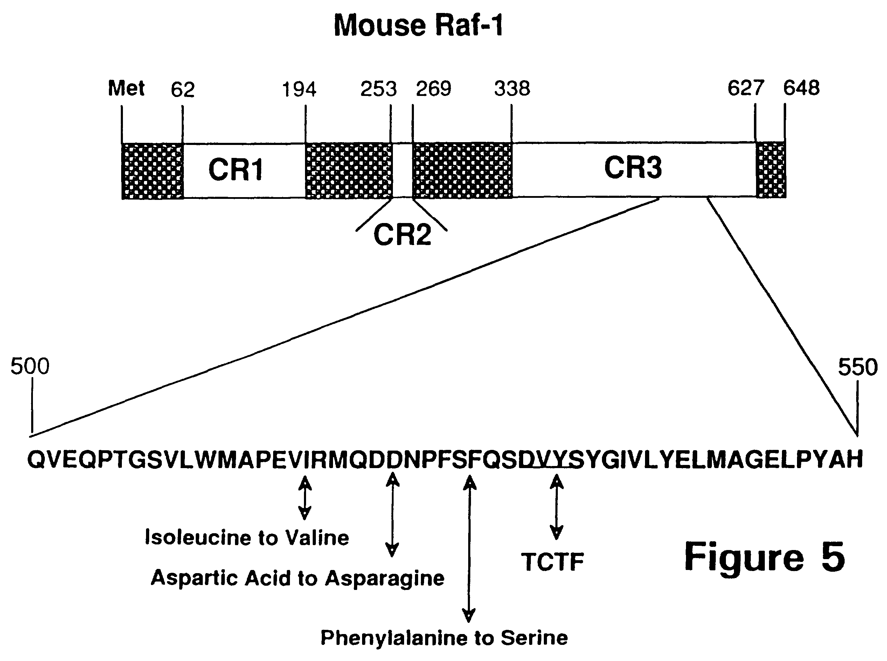

Figure 5. Schematic of Raf-1 protein showing sites of ENU induced mutations. CR1, CR2, and CR3 represent conserved regions 1, 2 and 3. cDNAs were made from tumor derived poly(A)+ RNA using MoMuLV reverse transcriptase. Primers (MR1 sequence and MR2 sequence) encompassing a 435 base pair region c-raf were then used to amplify this region via PCR. The amplification mixture was then run on 1.7% agarose gels and the 435 bp product isolated. This isolated fragment was then treated with T4 polymerase and cloned into the HincII site of M13mp18 for sequencing. Another set of primers (EMR1 sequence and EMR2 sequence) was designed containing EcoRI sites at the termini and used to amplify a 609 base pair region (encompassing the original 435 base pair region). Isolated products from these reactions were then digested with EcoRI and cloned into the EcoRI site of KS. Sequencing reactions were carried out using the Sequenase kit (USB) according to the recommended protocols for single and double stranded sequencing. Sequencing reactions were run on 6% polyacrylamide denaturing gels at 65 watts. Gels were vacuum dried at 80 degrees C and exposed to X-ray film. In each case a normal allele was also sequenced along with the mutant allele.

Figure 6. Schematic for Identifying c-raf-1 mutations. Primers 1 and 2 are shown in SEQ ID NO:8 and SEQ ID NO:9, respectively.

### DETAILED DESCRIPTION OF THE INVENTION

The present invention relates to methods that involve amplifying a region of the c-raf-1 gene (the sequence of a mouse c-raf-1 gene is shown in SEQ ID NO:10; the nucleotide and corresponding amino acid sequence of a human c-raf-1 gene is shown in SEQ ID NO:11 and SEQ ID NO:12, respectively).

The following Examples demonstrate consistent point mutations of the c-raf-1 proto-oncogene, within a small region of the kinase domain, in a mouse model for chemical tumor induction. This is the first demonstration of point mutated raf genes in vivo, and the first isolation of activating in vivo point mutations in the kinase domain of a proto-oncogene. The tumors examined show a selective specificity for Raf-1 mutations as another family of genes, the ras proto-oncogenes which are frequently activated by point mutation in both animal and human tumors (S. Rodenhuis et al., Am. Rev. Respir. Dis. 142, S27-30; T.R. Devereux et al., Carcinopenesis 12, 299 (1991)), is not involved.

The present invention is described in further detail in the following non-limiting examples.

### EXAMPLES

The following protocols and experimental details are referenced in the examples that follow:
RNA Isolation. Tumors were excised, a small portion minced in PBS (phosphate buffered saline solution) for passaging in nude mice, frozen immediately in a dry ice/ethanol bath, and stored at -70° until RNA extraction. Frozen tissues were minced on wet ice in a guanidine thiocyanate buffer (4M guanidine thiocyanate 10mM EDTA, 2% N-lauryl sarcosine, 2% beta-mercaptoethanol, 10mM Tris (pH=7.6)), disrupted in a Dounce homogenizer, and extracted three times with phenol: chloroform: isoamyl alcohol (24:24:2). Supernatants were then transferred to SW41 tubes, 100 µg of cesium chloride per ml was added to the supernatant which was then underlayed with one half saturated cesium chloride in 10mM EDTA (pH=7.0; index of refraction 1.3995-1.4000), and centrifuged at 25,000 rpm for 20 hours in a Sorvall SW-41TI rotor using a Beckman model L5-50 ultracentrifuge. Supernatants were removed and RNA pellets dissolved in 4 ml resuspension buffer (10 mM Tris-HCl pH=7.6, 5% beta-mercaptoethanol, 0.5% N-lauryl sarcosine, 10 mM EDTA), extracted once with phenol:chloroform:isoamyl alcohol, sodium acetate added to 0.12M and RNA precipatated with two volumes ethanol at-20°C overnight. Precipitates were centrifuged at 9,000 rpm in a Sorvall SS-34 rotor for 30 minutes, and pellets redissolved in RNA sample buffer (10 mM Tris pH=7.4, 1mM EDTA, 0.05% sodium dodecyl sulfate) and concentrations determined by absorbance at 260 nm. Poly (A)* RNA was isolated by binding to oligo dT cellulose columns in high salt (10mM Tris pH=7.4, 1mM EDTA, 0.05% SDS, 500mM NaCl), and eluting with RNA sample buffer heated to 40°C.
Northern Blotting. 5 µg poly(A)+ RNA per lane was ethanol precipitated, desiccated, resuspended in loading buffer (20mM MOPS pH=6.8, 5mM sodium acetate, 1mM EDTA, 50% formamide, 6% formaldehyde), heated at 65°C for 5 min., quick chilled on wet ice for 10 min., and electrophoresed through a 0.7% agarose gel containing 2.2 M formaldehyde, 20mM MOPS [pH=6.8], 5mM sodium acetate, and 1mM EDTA. Gels were then blotted overnight onto nitrocellulose filters via capillary transfer in 20X SSC, filters were washed in 3X SSC for 10 min. and baked at 80°C for 2 hours.
Hybridizations. Filters were prehybridized at 42°C in 5X SSC, 50% formamide, 20mM sodium phosphate pH=6.8, 200 µg/ml PVP-40, 200 µg/ml Ficoll® 400, 200 µg/ml bovine serum albumin, and 200 µg/ml sonicated sheared salmon sperm DNA. Blots were then hybridized with 500,000 cpm/ml of random primed ³²P labeled probes overnight at 42°C in prehybridization solution with 5% dextran sulfate. Blots were washed with agitation in 2X SSC, 0.1% SDS at room temperature six times for 20 minutes each wash, then washed once at 45°C in 0.1X SSC for 15 minutes. Filters were exposed to X-AR 5 film at -70°C.

### EXAMPLE 1

### Tumor Induction

NFS female mice were mated with AKR males and pregnant females given a transplacental injection of 1-ethyl-1-nitrosourea (ENU) at a dosage of 0.5 mM/Kg mother's body weight on day 16 of gestation, counting plug date as day one. ENU was chosen for tumor induction since it is a very potent direct acting carcinogen capable of modifying any base in vivo (Singer, B. et al., 1983. Molecular Biology of Mutagens and Carcinogens, Plenum Press, New York). ENU alkylates all tissues with roughly the same efficiency (E. Scherer et al., Cancer Lett. 46, 21 (1989)) and has a very short half life in vivo (E.M. Faustman et al., Teratology 40, 199 (1989)) allowing specific mutagenesis of tissues which are mitotically active at a particular time. NFS and AKR were chosen as parental strains based on earlier studies which showed them to be particularly susceptible to lung tumors following ENU exposure (B.A. Diwan et al., Cancer Res. 34, 764 (1974); S.L. Kauffman, JNCI 57, 821 (1976)). With this procedure nearly 100% of the offspring develop lung adenocarcinomas and approximately 70% develop, in addition, T-cell lymphomas with a mean latency of approximately 20 weeks. In order to achieve more rapid tumor development,weaning mice were treated with weekly injections of a tumor promoter, the antioxidant butylated hydroxytoluene or BHT (20mg/kg body weight dissolved in corn oil). BHT was used as it has been demonstrated to cause lung lesions and hyperplasia when injected into mice (A.A. Marino et al., Proc. Soc. Exp. Biol. Med. 140, 122 (1972); H. Witschi et al., Proc. Soc. Exp. Biol. Med., 147, 690 (1974); N. Ito et al., CRC Crit. Rev. Toxicol. 15, 109 (1984)). In the present system it nearly doubles the rate at which tumors develop. Figure 1 compares tumor induced mortality with age of animals for those receiving ENU alone, and those receiving ENU and promoted with BHT. These curves demonstrate that when BHT is given the mean age of tumor induced mortality decreases from approximately 20 weeks to around 12, and there is also a decrease in initial latency. These curves are significantly different with a confidence limit greater than 99.99% using a 2-tailed Cox test. In addition, BHT promotion, while increasing the rate at which tumors develop, does not affect the tumor spectrum.

### EXAMPLE 2

### Oncogene Expression

Northern blot analysis revealed elevated levels of c-raf-1, as compared to normal tissue, in every tumor examined (Figure 2), and Western blot analysis showed that protein levels correlated with message levels (U.R. Rapp et al., in Oncogenes and Cancer, S.A. Aaronson et al., Eds. (Tokyo/VNU Scientific Press, Tokyo, 1987) pp. 55-74). In addition, in cell lines derived from primary tumors, Raf-1 protein kinase activity was shown by immune-complex kinase assays to be constitutive. Further analysis of other oncogenes revealed no consistent pattern of expression except for ras and myc family genes. In the case of the myc family, one member (either c-, N-, or L-myc) was overexpressed but never more than one. For the ras genes, at least one member (Ki-, Ha-, or N-ras), and often more than one, was expressed at high levels when compared with the normal tissue. In addition all oncogenes examined via Northern analysis exhibited full length, normal sized transcripts.

ras genes were considered likely candidates for mutational activation since oncogenic forms of Ki-ras have previously been observed in lung tumors (S. Rodenhuis et al., Am. Rev. Respir. Dis. 142, S27-30; T.R. Devereux et al., Carcinogenesis 12, 299 (1991)) and ENU is a point mutagen (Singer, B. et al., 1983. Molecular Biology of Mutagens and Carcinogens, Plenum Press, New York). A systematic analysis of various ras codons known to be involved in oncogenic activation was therefore performed . Ha-, Ki-, and N- ras were examined at codons 12, 13, and 61 for potential mutations via RNAse protection assays (R.M. Myers et al., Science 230, 1242 (1985); E. Winter et al., Proc. Natl. Acad. Sci. USA 82, 7575 (1985)), PCR amplification followed by subsequent sequencing (F. Sanger et al., J. Mol. Biol. 13, 373 (1975)), and PCR amplification followed by diagnostic restriction digests (W. Jiang et al., Oncogene 4, 923 (1989)). PCR amplification creating diagnostic enzyme sites is a very efficient way of examining alleles for mutations at known sites and involves designing a PCR primer whose 3' end lies next to and produces a novel restriction site encompassing the codon of interest. Following amplification, PCR products from normal alleles will contain the new restriction site, while mutant alleles will not. Digestion of the product from tissue with two normal alleles results in all product being cut; however, if one allele contains a mutation, only half of the product will be digested. Figure 3 shows the results of amplification and diagnostic digestion applied to Ki-ras codon 12 in several tumors and cell lines. The first panel is from a set of lymphomas. F1 is DNA from a normal untreated mouse and both alleles are cut by BstNl, indicating the presence of two normal alleles. MCA5 is a murine cell line known to contain a Ki-ras codon 12 mutation (L.F. Parada et al., Mol. Cell. Biol. 3, 2298 (1983)), and only the amplified normal allele is cleaved. Of the five tumors shown in the second panel, one shows a mutant Ki-ras allele. The next panel shows some of the lung tumors tested and none of them exhibit a mutant allele, and the final panel shows tumor derived cell lines. The first three are from lymphomas and the last three from lung adenocarcinomas. One lung tumor line (#117) has a Ki-ras 12 mutation that was not present in the primary tumor but came up upon transplantation. This analysis has been performed with Ki, Ha and N-ras genes at codons 12 and 61. Of all the tumors and cell lines examined by this method for mutations of the three ras genes at codons 12 and 61, the two shown here were the only ones detected. Examination of codon 13 was done by PCR amplification of genomic DNA surrounding codon 13 followed by cloning into KS+ (Stratagene) and double stranded sequencing. Table I summarizes the ras mutation data. The most notable point from this table is the conspicuous lack of ras mutations in these tumors. In fact the number of ras mutations is much lower than would be expected for a sampling of spontaneous tumors (S. Rodenhuis et al., Am. Rev. Respir. Dis. 142, S27-30; T.R. Devereux et al., Carcinogenesis 12, 299 (1991); J.L. Bos, Cancer Res. 49, 4682 (1989)). Having eliminated ras genes as playing a primary role in the genesis of these ENU induced tumors, c-raf-1 was investigated for possible small or point mutations.

**TABLE I**

| Tumors and Cell Lines Positive for ras Mutations | | | | | | |
|---|---|---|---|---|---|---|
| | Codon 12 | | Codon 13 | | Codon 61 | |
| | Tumors | Cell Lines | Tumors | Cell Lines | Tumors | Cell Lines |
| Ha-ras | 0/10 | 0/6 | 0/6 | 0/2 | 0/10 | 0/6 |
| Ki/ras | 1*/10 | 1/6 | 0/6 | 0/2 | 0/6 | 0/2 |
| N-ras | 0/10 | 0/6 | 0/6 | 0/2 | 0/10 | 0/6 |

| | | | | | | |
|---|---|---|---|---|---|---|
| * This was a second passage tumor in which the original tumor did not contain a Ki-ras mutation. | | | | | | |

Table I: Summary of mutation analysis for Ha-, Ki-, and N-ras at codons 12, 13, and 61. Each box displays the number of mutations detected, over the number of tumors and tumor derived cell lines examined via RNAse protection, sequencing or diagnostic digestion, for each of the nine codons.

### EXAMPLE 3

### Mutations in Raf-1

Since no point mutations had been described for raf genes in vivo, as had been for the ras genes (E. Santos et al., Science 223, 661 (1984); S. Rodenhuis, N. Enal. J. Med. 317, 929 (1987); M. Barbacid, Eur. J. Clin. Invest. 20, 225 (1990); F. Sanger et al., J. Mol. Biol. 13, 373 (1975)), point mutations were screened for using RNAse protection assays (R.M. Myers et al., Science 230, 1242 (1985); E. Winter et al., Proc. Natl. Acad. Sci. USA 82, 7575 (1985)). Figure 4 shows a typical protection assay using a c-raf-1 probe. In this experiment the probe used covered the 3' end of raf-1 from the 3' most StuI site to the end of the coding sequence. The first lane is a marker (pBR322 digested with HaeIII), the second shows the probe alone undigested, the third lane shows the probe hybridized to unrelated RNA in this case tRNA, the fourth lane shows hybridization with v-raf transformed cells and the lower bands represent cleavage at points where the mouse c-raf-1 gene differs from v-raf. The fifth lane shows hybridization with RNA isolated from a normal lung of an untreated F1 mouse, the next lanes are RNA isolated from several tumors. In the case of the normal RNA, only one, fully protected, band is detected while in the case of the tumors two major bands are seen after digestion. 20 out of 20 tumors analyzed in this fashion showed this extra band. These data demonstrate the following major points: 1) there is a tumor specific alteration in c-raf-1 that results in a region of non-homology recognizable by either RNAse A or T1; 2) The alterations are confined to the same region of one allele as two bands of equal size are present in the tumor lanes, and; 3) both alleles were expressed at comparable levels as both bands are of approximately equal intensity. In the assay shown 5 µg of poly(A)+ RNA was hybridized from normal tissue, and 1 µg was used from the tumors. This was necessary to get signals that could be compared on the same gel due to the overexpression of c-raf-1 in the tumors. By running these assays with various markers it was possible to estimate the approximate site of the alteration(s) to be in the vicinity of the exon 14/exon 15 junction. In order to define the precise genetic alteration or alterations, PCR primers were designed which would generate a 600 bp fragment encompassing this region. cDNAs from tumor derived RNA were then amplified and cloned into KS+ (Stratagene) for double stranded sequencing. The sequencing results from several tumors are shown in Figure 5. The top portion of Figure 5 presents a cartoon of the mouse Raf-1 protein. There are three conserved regions CR1, CR2 and CR3 with CR3 representing the kinase domain. The probe used in the RNAse protection assays covers the indicated area, and the PCR primers amplified the bracketed region. Sequencing through this area revealed a variety of mutations just downstream of the APE site. These mutants are shown in an expanded version at the bottom of Figure 5 (See also SEQ ID NO:1 for normal mouse sequence and SEQ ID NO:2, 3, 4, and 5 for mutant sequences). These mutants were isolated from four separate tumors, and in each case a normal allele (SEQ ID NO:1) was also sequenced. Repeating the cDNA synthesis, PCR amplification, cloning and sequencing gives the same sequence and normal tissue shows no mutations demonstrating that these alterations are not artifactual. Sequence covering the amplified region has been examined and it is interesting that all of these changes occur within a very small region of the raf protein. In fact the region where these mutations occur overlaps an epitope shared by monoclonal antibodies generated against raf (W. Kolch et al., Oncogene 5, 713 (1990)), and computer modeling of the protein shows this to be a hydrophilic domain, the structure of which is predicted to be altered by these mutations. This indicates a biologically important region for the molecule and indeed the first of these mutation tested in NIH3T3 cell assays, after cloning into a retroviral expression vector (E1-neo, (G. Heidecker et al., Mol. Cell. Biol. 10, 2503 (1990))), was found to be weakly transforming when driven by a Moloney LTR. The transformation efficiency was comparable to EC2, a previously characterized mutation of human c-raf-1 cDNA (G. Heidecker et al., Mol. Cell. Biol. 10, 2503 (1990); C. Wasylyk et al., Mol. Cell. Biol. 9, 2247 (1989)) and ∼20 fold lower than the v-raf oncogene.

## Claims

1. A method of identifying the presence of a cancer in a subject, comprising detecting the presence of a point mutation in the c-raf-1 gene of the subject.

2. A method according to claim 1, wherein the mutation is in a region encoding amino-acids 514 to 535 of SEQ ID NO:12.

3. A method according to claim 1, wherein the mutation is in a region encoding amino-acids 500 to 550 of SEQ ID NO:12.

4. A method according to claim 1, wherein the mutation is in a region encoding amino-acids 450 to 630 of SEQ ID NO:12.

5. A method according to any preceding claim, wherein, prior to detection, said gene or said region is amplified, e.g. by the polymerase chain reaction (PCR).

6. A method according to claim 5, wherein the amplification is by employing a primer comprising SEQ ID NO:9 and a primer comprising SEQ ID NO:8.

7. A method according to claim 5 or claim 6, wherein the detection comprises analysis of the amplification products by DNA sequencing.

8. A method according to any preceding claim, wherein the cancer is a lung adenocarcinoma.

9. Nucleic acid primers which can specifically hybridize to the nucleic acid sequence encoding a mutation in amino-acids as defined in any of claims 2 to 4.

## Patentansprüche

1. Methode zum Feststellen des Vorliegens von Krebs in einem Subjekt, umfassend den Nachweis des Vorliegens einer Punktmutation in dem c-raf-1-Gen des Subjekts.

2. Methode nach Anspruch 1, worin die Mutation in einem Bereich liegt, der die Aminosäuren 514 bis 535 der SEQ ID-Nr: 12 kodiert.

3. Methode nach Anspruch 1, worin die Mutation in einem Bereich liegt, der die Aminosäuren 500 bis 550 der SEQ ID Nr: 12 kodiert.

4. Methode nach Anspruch 1, worin die Mutation in einem Bereich liegt, der die Aminosäuren 450 bis 630 der SEQ ID Nr: 12 kodiert.

5. Methode nach irgendeinem vorangehenden Anspruch, worin das Gen oder der Bereich vor dem Nachweis amplifiziert werden, z.B. durch Polymerase-Kettenreaktion (PCR).

6. Methode nach Anspruch 5, worin die Amplifizierung unter Anwendung eines Primers, der SEQ ID Nr:9 umfaßt, und eines Primer, der SEQ ID Nr:8 umfaßt, erfolgt.

7. Methode nach Anspruch 5 oder Anspruch 6, worin der Nachweis die Analyse der Amplifizierungsprodukte durch DNA-Sequenzierung umfaßt.

8. Methode nach irgendeinem vorangehenden Anspruch, worin der Krebs ein Lungen-Adenocarcinom ist.

9. Nucleinsäure-Primer, die spezifisch an eine Nucleinsäuresequenz hybridisieren können, welche eine Mutation von Aminosäuren, wie in irgendeinem der Ansprüche 2 bis 4 definiert, kodiert.

## Revendications

1. Procédé pour identifier la présence d'un cancer chez un sujet, comprenant la détection de la présence d'une mutation ponctuelle dans le gène c-raf-1 du sujet.

2. Procédé suivant la revendication 1, dans lequel la mutation est située dans une région codant pour les aminoacides 514 à 535 de la SEQ ID N° 12.

3. Procédé suivant la revendication 1, dans lequel la mutation est située dans une région codant pour les aminoacides 500 à 550 de la SEQ ID N° 12.

4. Procédé suivant la revendication 1, dans lequel la mutation est située dans une région codant pour les aminoacides 450 à 630 de la SEQ ID N° 12.

5. Procédé suivant l'une quelconque des revendications précédentes, dans lequel, avant la détection, ledit gène ou ladite région est amplifié, par exemple par la réaction en chaîne avec une polymérase (PCR).

6. Procédé suivant la revendication 5, dans lequel l'amplification est effectuée en utilisant une amorce comprenant la SEQ ID N° 9 et une amorce comprenant la SEQ ID N° 8.

7. Procédé suivant la revendication 5 ou la revendication 6, dans lequel la détection comprend l'analyse des produits d'amplification par séquençage d'ADN.

8. Procédé suivant l'une quelconque des revendications précédentes, dans lequel le cancer est un adénocarcinome pulmonaire.

9. Amorces d'acide nucléique qui peuvent s'hybrider spécifiquement à la séquence d'acide nucléique codant pour une mutation dans des aminoacides définis dans l'une quelconque des revendications 2 à 4.
